# EUROPÄISCHE PATENTANMELDUNG

(11) **EP 1 911 479 A1**
(43) Veröffentlichungstag der Anmeldung: **16.04.2008**
(21) Anmeldenummer: 06121981.2
(22) Anmeldetag: 09.10.2006
(51) Int. Cl.: A61M 5/315, A61M 5/145

(54) **Verabreichungsvorrichtung mit magnetischer Verbindung zwischen Kolben und Kolbenstange**

(71) Anmelder: F.HOFFMANN-LA ROCHE AG, 4070 Basel (CH); Roche Diagnostics GmbH, 68305 Mannheim (DE)
(72) Erfinder: Friedli, Kurt, 3421 Lyssach (CH)
(74) Vertreter: Schwabe - Sandmair - Marx

(57) **Zusammenfassung**

Die Erfindung betrifft eine Verabreichungsvorrichtung zur Verabreichung eines Produktes, das in den Körper eines Patienten einzubringen ist, mit einem Aufnahmevolumen für das Produkt und mit einem Kolben, der in dem Aufnahmevolumen verschiebbar ist, um das Produkt aus dem Aufnahmevolumen (2) auszulassen, und mit einer Kolbenstange (6a), die mit dem Kolben (3) in Wirkverbindung bringbar ist, wobei erfindungsgemäß der Kolben (3) eine eine magnetische Wirkung erzeugende erste Einrichtung (15) umfasst, während die Kolbenstange (6a) eine eine magnetische Wirkung aufweisende zweite Einrichtung (16) umfasst, wobei der Kolben (3) und die Kolbenstange (6a) mittels der ersten und der zweiten Einrichtung (15, 16) miteinander verbindbar sind.

## Beschreibung

Die Erfindung betrifft eine Verabreichungsvorrichtung zur Verabreichung eines Produktes, das in den Körper eines Patienten einzubringen ist, mit einem Aufnahmevolumen für das Produkt und mit einem Kolben, der in dem Aufnahmevolumen verschiebbar ist, um das Produkt aus dem Aufnahmevolumen auszustoßen, und mit einer Kolbenstange, die mit dem Kolben in Wirkverbindung bringbar ist, gemäß dem Oberbegriff des Anspruchs 1.

Vorrichtungen gemäß dem Oberbegriff des Anspruchs 1 werden als Infusions- oder Injektionsvorrichtungen zur dosierten Verabreichung eines flüssigen Produkts, also beispielsweise eines Medikaments, von Insulin oder dergleichen, eingesetzt. Dabei wird das Aufnahmevolumen als Reservoir für den zu verabreichenden medizinischen oder therapeutischen Wirkstoff verwendet. Bekanntermaßen wird der Kolben von der Kolbenstange mittels eines Antriebsmechanismus axial zu dem Auslass des Aufnahmevolumens verschoben, um das Produkt aus dem Aufnahmevolumen auszustoßen. Das Aufnahmevolumen ist herkömmlicherweise in der Regel austauschbar. Dementsprechend ist es erforderlich, zwischen dem Kolben und der Kolbenstange eine lösbare Verbindung bereitzustellen. Da jedoch nicht nur ein Ausstoßen des Wirkstoffes vonnöten ist, sondern zudem noch eine sichere und genaue Dosierung des Wirkstoffes bzw. Produkts, muss die Verbindung zwischen Kolben und Kolbenstange so ausgebildet sein, dass eine axiale Verstellung des Kolbens relativ zur Kolbenstange nicht möglich ist.

Bekanntermaßen wird die Kolbenstange mit einem Gewinde ausgebildet und in Eingriff mit einem Gegengewinde im Kolben gebracht. Somit kann durch Verschrauben des Kolbens mit der Kolbenstange eine Verbindung bewerkstelligt werden. Umgekehrt kann durch Aufhebung der Verschraubung die Verbindung zwischen Kolben und Kolbenstange wieder gelöst werden. Zu Problemen kommt es, wenn beim Aufschrauben oder Abschrauben die Kolbenstange und der Kolben eine Positionsdifferenz aufweisen. Ist eine solche Positionsdifferenz beim Aufschrauben vorhanden, kann Luft entweder in das Aufnahmevolumen eingesaugt werden oder es kann ein Teilvolumen des Produkts im Aufnahmevolumen unbeabsichtigt ausgeschüttet werden. Eine andere Verbindung wird durch eine Schnappverbindung bewerkstelligt.

Insoweit die Kolbenverstellung durch eine radiale Verstellung des Kolbens bzw. der Kolbenstange erfolgt, d.h., die Kolbenstange ist beispielsweise eine Gewindestange, die zur Ausschüttung des Produktes bzw. zum Rückziehen des Kolbens bzw. der Kolbenstange in Rotation versetzt wird, tritt eine große Reibung auf, die zu überwinden ist, wobei die hierfür erforderlichen Kräfte ebenfalls zu Dosierproblemen führen können.

Wie aufgezeigt, sind beim Stand der Technik insoweit Nachteile zu veranschlagen, als eine Positionsdifferenz, die relativ regelmäßig vorkommt, und die zwischen dem Kolben und der Kolbenstange auftritt, zu den oben erläuterten Nachteilen führt.

Dementsprechend ist es eine Aufgabe gemäß der Erfindung, eine verbesserte Verbindungstechnologie bereitzustellen, die den Nachteilen im Stand der Technik soweit als möglich Abhilfe verschafft.

Insbesondere soll eine im Wesentlichen spielfreie bzw. positionsdifferenzfreie Verbindungstechnologie bereitgestellt werden.

Gemäß der Erfindung wird diese Aufgabe durch eine Verabreichungsvorrichtung mit den Merkmalen im Anspruch 1 gelöst. Zweckmäßige Ausführungsformen gemäß der Erfindung gehen aus den Unteransprüchen hervor.

Die mit der Verabreichungsvorrichtung gemäß der Erfindung erzielbaren Vorteile beruhen darauf, dass der Kolben eine eine elektromagnetische oder magnetische Wirkung erzeugende erste Einrichtung aufweist, wobei die Kolbenstange eine eine elektromagnetische oder magnetische Wirkung erzeugende zweite Einrichtung aufweist, und wobei der Kolben und die Kolbenstange mittels der ersten und der zweiten Einrichtung miteinander verbindbar sind. Mit anderen Worten, gemäß der Erfindung ist es möglich, eine nahezu spielfreie bzw. vollkommen spielfreie Verbindung zwischen der Kolbenstange und dem Kolben herzustellen. Die Verbindung kann auch wieder ohne ein Spiel getrennt werden. Hier ist weder zu befürchten, dass Luft eintritt, noch dass zu verabreichendes Produkt unbeabsichtigterweise austritt, da Positionsdifferenzen zwischen der Kolbenstange und dem Kolben bei der Verbindung bzw. beim Lösen der Verbindung nicht auftreten können.

Vorteilhafterweise ist die erste Einrichtung aus einem magnetisierbaren Material, einem permanent magnetischen Material oder es kann auch ein eine elektromagnetische Wirkung aufbringendes induktives Element vorgesehen sein. Prinzipiell reicht es aus, wenn es sich bei der ersten Einrichtung um einen Block magnetisierbaren Materials handelt, etwa Eisen oder einem anderen ferromagnetischen Material. Auch ein schwach permanent magnetisches Material kann zum Einsatz gelangen. Die Einbringung eines eine elektromagnetische Wirkung aufbringenden induktiven Elements, etwa einer Spule, dürft in Bezug auf den Kolben nicht ganz trivial sein, da Zuleitungen erforderlich sind, oder aber eine Induktion erforderlich wäre, d.h., permanent ein Wechselfeld anliegen müsste, um der Spule eine elektromagnetische und damit magnetische Wirkung zu verleihen.

Entsprechend kann die zweite Einrichtung, abhängig von der Ausgestaltung der ersten Einrichtung, eine magnetisierbares Material, ein permanent magnetisches Material oder ein eine elektromagnetische Wirkung aufbringendes induktives Element umfassen. Bevorzugt wird hier ein permanent magnetisches Material, etwa magnetisiertes Eisen oder eine magnetisierte Legierung. Natürlich kann auch ein eine elektromagnetische Wirkung aufbringendes induktives Element etwa in der Form einer Spule hier vorgesehen werden. Jedoch kann die erforderliche dauerhafte Bereitstellung eines elektromagnetischen Feldes Probleme bereiten, so dass sich je nach Ausgestaltung der ersten Einrichtung entweder ein magnetisierbares Material oder ein permanent magnetisches Material für die zweite Einrichtung an der Kolbenstange anbietet.

Dementsprechend ist es vorteilhaft, dass, wenn die erste Einrichtung ein magnetisierbares Material umfasst, die zweite Einrichtung aus einem permanent magnetischen Material aufgebaut ist, und umgekehrt, um eine verbindende Wirkung zwischen der ersten und der zweiten Einrichtung bereitstellen zu können, ohne dass ein elektrisches Feld angelegt werden muss.

Um die Lage der ersten bzw. der zweiten Einrichtung relativ zu dem Aufnahmevolumen und damit den Füllstand des Aufnahmevolumens ermitteln zu können, ist es vorteilhaft, wenn die Lage der ersten und/oder der zweiten Einrichtung relativ zu dem bzw. in dem Aufnahmevolumen erfassbar ist. Zu diesem Zweck kann vorteilhafterweise ein Sensor vorgesehen werden, der die Lage der ersten und/oder der zweiten Einrichtung im Aufnahmevolumen erkennbar oder messbar macht. Vorteilhafterweise kann der Sensor aufgrund einer magnetischen Wechselwirkung zwischen der ersten und/oder der zweiten Einrichtung und dem Sensor die Position der ersten und/oder der zweiten Einrichtung feststellen und somit den Füllstand des Produktes im Aufnahmevolumen ermitteln. Entsprechend kann auch die Bewegungsstrecke ermittelt werden, um die die erste und/oder die zweite Einrichtung mittels der Kolbenstange voranbewegt werden, da der Sensor zur Positionsfeststellung die Position insbesondere aufgrund von Positionsänderungen feststellen kann.

Besonders vorteilhaft ist es, wenn der Sensor ein permanent magnetischer, berührungsfreier Ortsveränderungssensor ist, der die Position der ersten und/oder der zweiten Einrichtung über die Änderungen von deren Positionen erfasst.

Natürlich ist es auch möglich, hier andere Sensoreinrichtungen zu verwenden, beispielsweise eine Zeile mit fotoempfindlichen Elementen, die aufgrund von Reflektionswerten der Wandung des Volumens mit oder ohne Kolben und/oder Kolbenstange die Position des Kolbens innerhalb des Aufnahmevolumens und damit die Produktmenge bzw. die Produktmengenänderung im Betrieb der erfindungsgemäßen Verabreichungsvorrichtung ermitteln kann. Dabei kann beispielsweise der Kolben auch aus einem hellen Material ausgebildet sein, wobei ein äußerer umfänglicher Bereich schwarz eingefärbt sein kann, um diese Kontraständerung bei Bewegungen für eine auf lichtempfindlichen Sensorelementen basierende Positionsbestimmung abzustimmen.

Zwar ist es auch möglich, die Kolbenstange vom Kolben durch eine hohe Beschleunigung, etwa einen Ruck, zu trennen, jedoch ist es vorteilhafter, wenn eine elektromagnetische Anordnung vorgesehen ist, mit der ein magnetisches Gegenfeld aufbaubar ist. Mit diesem elektrischen Gegenfeld kann die Verbindung der ersten mit der zweiten Einrichtung aufgehoben werden, um ohne jedes Spiel den Kolben von der Kolbenstange trennen zu können und ein neues Aufnahmevolumen in die erfindungsgemäße Verabreichungsvorrichtung einzusetzen.

Die elektromagnetische Anordnung zum Aufbau eines magnetischen Gegenfeldes oder Neutralisationsfeldes kann dabei als Teil des Sensors ausgebildet sein, der ebenfalls eine Spule benötigt. Dies geht natürlich nur, solange das magnetische Gegen- bzw. Neutralisationsfeld relativ gering ist. Sollten höhere magnetische Kräfte erforderlich sein bzw. sollte ein stärkeres magnetisches Feld erforderlich sein, wird es nötig sein, eine separate elektromagnetische Anordnung zur Erzeugung eines magnetisches Gegen- bzw. Neutralisationsfeldes beispielsweise in der Form von Wicklungen vorzusehen. Diese Wicklungen können um das Aufnahmevolumen herumgelegt sein und/oder sie können um die zweite Einrichtung herum vorgesehen sein.

Vorteilhafterweise umfasst der Kolben der erfindungsgemäßen Vorrichtung eine Ausnehmung, die die erste Einrichtung wenigstens teilweise, vorzugsweise vollständig aufnimmt. Die erste Einrichtung weist auf ihre der zweiten Einrichtung zugewandten Seite eine Ebene Fläche auf, so dass beim Zusammenführen der ersten mit der zweiten Einrichtung keine Ortsdiskrepanzen auftreten können.

Vorteilhafterweise kann die erfindungsgemäße Vorrichtung mit einem Kraftsensor ausgebildet sein, der in Axialrichtung der Kolbenstange misst. Dieser Kraftsensor kann dann eingesetzt werden, um festzustellen, wann eine Verbindung zwischen der Kolbenstange und dem Kolben hergestellt ist. Ferner kann der Kraftsensor eingesetzt werden, um die Vortriebskraft zu messen, um auf diese Weise zu bestimmen, ob und wann der Kolben im Aufnahmevolumen soweit bewegt worden ist, dass die zu entlüftenden und mit Produkt zu befüllenden Bestandteile der erfindungsgemäßen Vorrichtung ordnungsgemäß mit Produkt befüllt sind bzw. entlüftet sind. Hierzu kann einerseits die vom Positionssensor gemessene Position der ersten und/oder der zweiten Einrichtung bzw. die Positionsänderung, und andererseits die Kraft gemessen werden, die für eine axiale Verschiebung der Kolbenstange erforderlich ist, da diese maximal werden sollte, wenn sämtliche mit Produkt zu befüllende Hohlräume der erfindungsgemäßen Vorrichtung entlüftet sind, d.h., mit Produkt befüllt sind.

Natürlich kann anstelle der Kolbenstange mit dem daran festgelegten Kolben auch das Aufnahmevolumen mit dem darin befindlichen Produkt relativ zu dem Kolben mit der daran festgelegten Kolbenstange verschoben werden. Hierbei müsste dann ein Positionssensor oder Positionsänderungssensor mit dem Aufnahmevolumen gekoppelt sein, um einen Messwert für eine Lageänderung des Kolbens relativ zu dem Sensor bereitstellen zu können. Dies würde einen Messwert ergeben, der eine Volumenänderung des Aufnahmevolumens erkennen lässt. In einem solchen Fall müsste der Antrieb gegebenenfalls in Verbindung mit dem Kraftsensor an dem Aufnahmevolumen angreifen, das zusammen mit dem Sensor vom Antrieb bewegt werden müsste. Sollte anstelle des Kolbens das Aufnahmevolumen, wie aufgeführt, bewegbar sein, wäre dies eine gleichwirkende und damit äquivalente Ausführungsform.

Nachfolgend wird die vorliegende Erfindung anhand der beigefügten Darstellungen näher erläutert, wobei weitere Vorteile, Zielsetzungen sowie Merkmale gemäß der Erfindung offenbart werden. In den Darstellungen zeigen:
- Figur 1: eine Prinzipdarstellung einer Verabreichungsvorrichtung mit Merkmalen gemäß der Erfindung in einer Schnittdarstellung;
- Figur 2: ein Aufnahmevolumen zur Verwendung mit einer Vorrichtung gemäß der Erfindung;
- Figur 3: ein Aufnahmevolumen zur Verwendung mit der Erfindung nach Figur 2 nach einem Montageschritt, der in Figur 2 angedeutet ist, in einer längsschnittlichen Darstellung; und
- Figur 4: eine weitere Ausführungsform gemäß der Erfindung in einer Prinzipdarstellung im Längsschnitt.

In den Figuren sind gleiche oder wenigstens funktionsgleiche Bestandteile mit gleichen bzw. einander entsprechenden Bezugsziffern benannt, so dass sich in der Regel eine mehrfache Erörterung gleicher Bestandteile erübrigt.

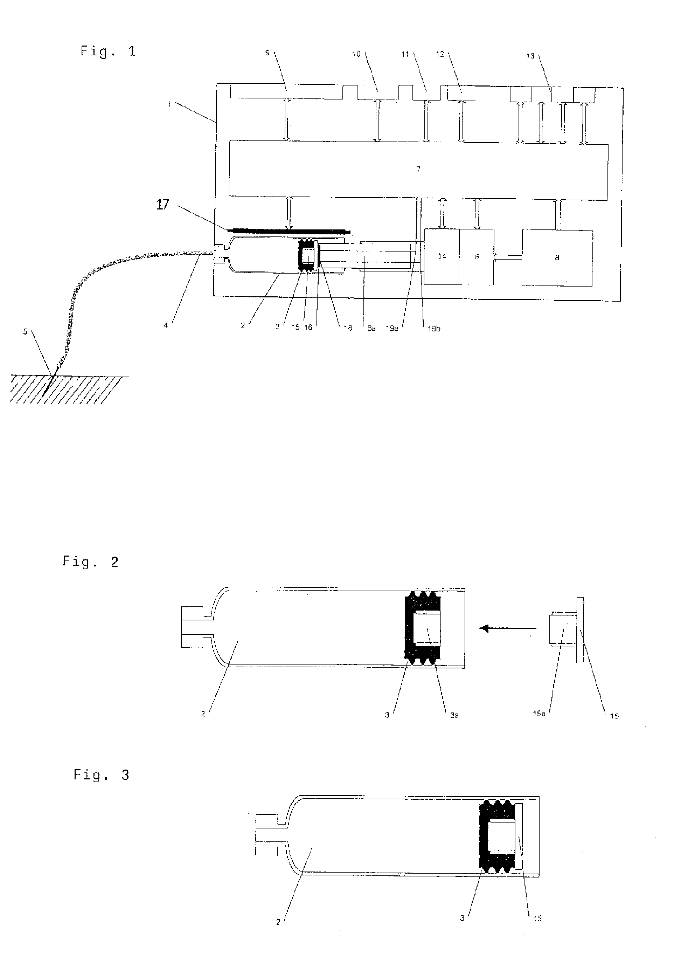

In Figur 1 wird eine Verabreichungsvorrichtung mit Merkmalen gemäß der Erfindung vorgestellt. Die Vorrichtung umfasst ein Gehäuse 1 in dem ein Aufnahmevolumen 2 angeordnet ist. Das Aufnahmevolumen 2 enthält ein Produkt, das einem Patienten zu verabreichen ist. Das Produkt im Aufnahmevolumen 2 kann über einen Kolben 3 aus dem Aufnahmevolumen 2 ausgestoßen werden. Es fliest aus dem Aufnahmevolumen 2 in einen Katheter 4, der über eine Infusionsnadel 5 das Produkt in den Körper des Patienten einführt.

Auf den Kolben 3 wird mittels einer Kolbenstange 6a eingewirkt, um das Produkt auszustoßen. Ein Antrieb 6 bewegt die Kolbenstange voran, wobei der Antrieb 6 mittels einer Energiequelle 8 versorgt werden kann.

Gemäß der Erfindung ist wesentlich, dass in den Kolben 3 eine erste Einrichtung 15 eingebracht ist, die eine elektromagnetische Wirkung entfalten kann. Die Kolbenstange 6a ist mit einer zweiten Einrichtung 16 ausgestattet, die ebenfalls eine magnetische oder elektromagnetische Wirkung entfalten kann.

Beispielhaft ist die erste Einrichtung 15 als ein Stück magnetisierbares Material, etwa Eisen oder dergleichen, bzw. eine magnetisierbare Legierung, die in den Kolben 3 eingeschraubt, eingeklebt oder eingepresst sein kann, ausgebildet. Die Art der Festlegung der ersten Einrichtung 15, nachfolgend als Koppelelement bezeichnet, ist dabei nicht wesentlich. Das Koppelelement 15 kann fix im Kolben angeordnet sein oder kann reversibel angeordnet sein. Im ersteren Fall kann das Koppelelement 15 ausgetauscht werden, wenn das Aufnahmevolumen 2 eventuell mit Kolben 3 nach Entleerung entsorgt wird und im anderen Falle kann das Koppelelement 15 bei Entsorgung des Aufnahmevolumens mit Kolben 3 ausgebaut und weiterverwendet werden.

Bei der beispielhaft dargestellten Ausführungsform gemäß Figur 1 ist die zweite Einrichtung 16 als Permanentmagnet ausgebildet. Nachfolgend wird die zweite Einrichtung 16 als Permanentmagnet 16 benannt werden, während in der Praxis natürlich auch das Koppelelement 15 als Permanentmagnet ausgebildet sein könnte, während der Permanentmagnet 16 aus einem magnetisierbaren Material sein könnte. Wesentlich ist nur die Verbindung zwischen dem Kolben 3 und der Kolbenstange 6a, die entgegen üblichen Verfahrensweisen nicht mechanisch sondern magnetisch bzw. elektromagnetisch erfolgt.

Zur Feststellung der Position des Koppelelements 15 und/oder des Permanentmagneten 16 und damit zur Feststellung der Position des Kolbens 3 innerhalb des Aufnahmevolumens 2 ist ein Sensor 17 vorgesehen. In einem bevorzugten Fall kann der Sensor 17 einen PLCD-Wegsensor sein, wie er beispielsweise von der Firma Tyco Electronics AMP GmbH verfügbar ist und in einem 2001 veröffentlichten Prospekt in seiner Funktion und bezüglich seiner Komponenten beschrieben worden ist. Dieser Prospekt sei hiermit auch in die vorliegende Offenbarung durch Bezugnahme aufgenommen. Die genannten Bezeichnungen sind nur zur technischen Information und nicht zu Wettbewerbszwecken aufgeführt.

Natürlich kann der Sensor 17 auch etwa in der Form einer Zeile von lichtempfindlichen Zellen vorgesehen sein, die eine Positionsangabe für den Kolben 3 durch unterschiedliche Lichtreflektionswerte oder Lichttransmissionswerte vornehmen kann.

Bei der vorgestellten Ausführungsform ist um den Permanentmagneten 16 ein Elektromagnet 18 angeordnet, d.h., stromführende Wicklungen sind um den Permanentmagneten 16 herumgewickelt. Eine Zu- und eine Ableitung 19a, 19b ist zur Versorgung des Elektromagneten 18 vorgesehen. Der Elektromagnet kann mit Strom beaufschlagt werden, um ein Neutralisierungsfeld oder Gegenfeld magnetischer Art aufzubauen, um die Verbindung zwischen dem Koppelelement 15 und dem Permanentmagneten 16 aufzuheben. Beispielsweise kann dieses Gegen- bzw. Neutralisationsfeld angelegt werden, wenn eine Verbindung zwischen dem Koppelelement 15 und dem Permanentmagneten 16 und damit zwischen den Kolben 3 und der Kolbenstange 6a aufgehoben werden soll. Umgekehrt kann auch ein kraftfreies Gegenfahren der Kolbenstange 6a gegen den Kolben 3 gewünscht sein, so dass beim Verbindungsvorgang ebenfalls das Anlegen eines Gegen- bzw. Neutralisationsmagnetfeldes hilfreich sein kann.

Zwischen der Kolbenstange 6a und dem Antrieb 6, der die Kolbenstange 6a vorantreibt oder zurückzieht, kann ein Kraftsensor 14 angeordnet sein. Mit diesem Kraftsensor 14 kann die beim Vorschub der Kolbenstange bzw. beim Zurückziehen der Kolbenstange 6a vom Antrieb 6 aufgebrachte Kraft gemessen werden, beispielsweise um zu messen, wann der Katheter 4 in Verbindung mit der Infusionsnadel 5 entlüftet ist, d.h., mit dem zu verabreichenden Produkt befüllt ist. Auch andere Zustände der erfindungsgemäßen Vorrichtung können über den Kraftaufwand bzw. den Verlauf des Kraftaufwandes beim Vortrieb der Kolbenstange 6a erfasst werden. Die hier aufgenommenen Messwerte können etwa auch in Kombination mit Positionswerten, die über den Sensor 17 aufgenommen werden können, Aufschluss über den Zustand der erfindungsgemäßen Vorrichtung geben.

Verschiedene Bestandteile der erfindungsgemäßen Vorrichtung können über eine elektronische Steuerung 7 überwacht und gesteuert werden. So kann die Steuerung 7 Messwerte von dem Sensor bzw. PLCD-Sensor 17 aufnehmen, sowie Messwerte von dem Kraftsensor 14 erhalten. Die Messwerte können für die Dosierung bzw. die Entlüftung und für die Berechnung des Füllstandes des Produkts im Aufnahmevolumen 2 herangezogen werden. Eine Energiequelle 8, die zur Versorgung der Steuerung 7 und des Antriebs 6 sowie weiterer Bestandteile der erfindungsgemäßen Vorrichtung herangezogen werden kann, kann in das Gehäuse 1 integriert sein.

Ansonsten kann die Vorrichtung gemäß der Erfindung mit üblichen Bedienungs-, Überwachungs- und Kommunikationselementen versehen sein, wie etwa einer Anzeige 9, einer IR-RF Kommunikationsschnittstelle 10, einem akustischen Signalgeber 11, einem taktelen Signalgeber 12 sowie einem Tastenfeld 13 zur Eingabe von Steuerbefehlen für die Steuerung 7.

Gemäß Figur 2 kann der Kolben 3 mit einer Aufnahmeausnehmung 3a ausgebildet sein, um einen korrespondierenden Einsetzabschnitt 15a des Koppelelements 15 aufzunehmen. Dem Einsetzabschnitt 15a gegenüber liegt ein Verbindungsabschnitt, der wenigstens auf der dem Ende der Kolbenstange 6a gegenüberliegenden Seite im Wesentlichen oder vollständig eben ausgebildet sein sollte. Wie Figur 2 zu entnehmen, ist das Koppelelement 15 in die Aufnahmeausnehmung 3a einzufügen, um den in Figur 3 dargestellten montierten Zustand zu erzielen, der für eine Verbindung zwischen dem Kolben 3 und der Kolbenstange 6a von grundlegender Bedeutung ist.

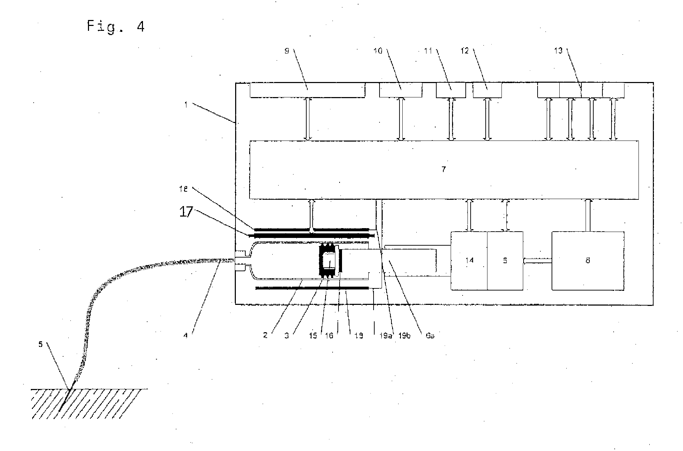

In Figur 4 ist eine weitere Ausführungsform mit Merkmalen gemäß der Erfindung dargestellte. Dabei ist abweichend von der Darstellung gemäß Figur 1 ein Elektromagnet 18 vorgesehen, der um das Aufnahmevolumen 2 insgesamt angeordnet ist, während dieser Elektromagnet 18 gemäß Figur 1 nur um den Permanentmagneten 16 selbst angeordnet ist. Bei der Ausführungsform gemäß Figur 4 ist es jedoch unproblematischer, Zuleitungen 19a, 19b vorzusehen, die den Elektromagneten 18 mit dem nötigen Strom versorgen.

Zu den Dimensionierungen der ersten und der zweiten Einrichtung 15, 16 gemäß der Erfindung ist festzuhalten, dass diese bevorzugt so ausgelegt sein können, dass etwa der Permanentmagnet 16 die maximal zu erwartenden axialen Kräfte, die beispielsweise durch einen Druckunterschied zwischen dem Aufnahmevolumen 2 und der Umgebung des Aufnahmevolumens 2 hervorgerufen wird, derart ausgleicht, dass die axiale Verbindung zwischen dem Kolben 3 und der Kolbenstange 6a hierdurch nicht gelöst werden kann.

Der bei beiden Ausführungsformen vorteilhaft vorgesehene Kraftsensor 14 ermöglicht es auch, eine Überwachung sicherzustellen, ob zwischen dem Kolben 3 und der Kolbenstange 6a die gewünschte und erwartete Verbindung zustande gekommen ist. Sobald die Kolbenstange 6a den Kolben 3 berührt, steigt die Vortriebskraft an, da eine Reibung und ein Fließwiderstand für das Produkt im Katheter 4 sowie in der Infusionsnadel 5 zu überwinden ist. Eine solche Messung kann gemäß einem Aspekt der Erfindung nunmehr automatisch durchgeführt werden, lediglich und zuverlässig überwacht durch die Steuerung 7.

### Bezugszeichenliste

- 1: Gehäuse
- 2: Reservoir, Aufnahmevolumen
- 3: Kolben
- 3a: Aufnahmeausnehmung
- 4: Katheter
- 5: Infusionsnadel
- 6: Antrieb
- 6a: Kolbenstange
- 7: Steuerung
- 8: Energiequelle
- 9: Anzeige
- 10: IR/RF Kommunikationsschnittstelle
- 11: Akustischer Signalgeber
- 12: Taktiler Signalgeber
- 13: Tastenfeld
- 14: Kraftsensor
- 15: Koppelelement, erste Einrichtung
- 15a: Einsetzabschnitt
- 16: Permanentmagnet, zweite Einrichtung
- 17: Sensor, PLCD-Sensor
- 18: Elektromagnet
- 19a: Stromversorgung Elektromagnet
- 19b: Stromversorgung Elektromagnet

## Patentansprüche

1. Verabreichungsvorrichtung zur Verabreichung eines Produktes, das in den Körper eines Patienten einzubringen ist, mit einem Aufnahmevolumen (2) für das Produkt und mit einem Kolben (3), der in dem und/oder relativ zum Aufnahmevolumen (2) verschiebbar ist, um das Produkt aus dem Aufnahmevolumen auszustoßen, und mit einer Kolbenstange (6a), die mit dem Kolben in Wirkverbindung bringbar ist, **dadurch gekennzeichnet,**
- **dass** der Kolben (3) eine eine elektromagnetische oder magnetische Wirkung erzeugende erste Einrichtung (15) aufweist, und
- **dass** die Kolbenstange (6a) eine eine elektromagnetische oder magnetische Wirkung erzeugende zweite Einrichtung (16) aufweist, wobei
- der Kolben (3) und die Kolbenstange (6a) mittels der ersten und der zweiten Einrichtung (15, 16) miteinander verbindbar sind.

2. Verabreichungsvorrichtung nach Anspruch 1, **dadurch gekennzeichnet, dass** die erste Einrichtung (15) ein magnetisierbares Material, ein permanent magnetisches Material oder ein eine elektromagnetische Wirkung aufbringendes induktives Element umfasst.

3. Verabreichungsvorrichtung nach einem der Ansprüche 1 oder 2, **dadurch gekennzeichnet, dass** die zweite Einrichtung (16) ein magnetisierbares Material, ein permanent magnetisches Material oder ein eine elektromagnetische Kraft aufbringendes induktives Element umfasst.

4. Verabreichungsvorrichtung nach einem der Ansprüche 1 bis 3, **dadurch gekennzeichnet, dass** relativ zu der in der Verabreichungsvorrichtung von der ersten und/oder der zweiten Einrichtung relativ zu dem Aufnahmevolumen (2) zurückzulegende Strecke wenigstens ein vorzugsweise auf eine magnetische Wechselwirkung reagierender oder auf eine optische Wechselwirkung reagierender Sensor (17) vorgesehen ist.

5. Verabreichungsvorrichtung nach Anspruch 4, **dadurch gekennzeichnet, dass** der Sensor ein permanent magnetischer, berührungsfreier Ortsveränderungssensor ist.

6. Verabreichungsvorrichtung nach einem der Ansprüche 1 bis 5, **dadurch gekennzeichnet, dass** eine elektromagnetische Anordnung (18) vorgesehen ist, mit der ein magnetisches Gegen- oder Neutralisationsfeld aufbaubar ist, um die Verbindung der ersten mit der zweiten Einrichtung (15, 16) aufzuheben.

7. Verabreichungsvorrichtung nach Anspruch 6 unter Rückbezug auf Anspruch 5, **dadurch gekennzeichnet, dass** der Sensor die elektromagnetische Anordnung umfasst oder dieser wenigstens teilweise entspricht.

8. Verabreichungsvorrichtung nach Anspruch 6, **dadurch gekennzeichnet, dass** die elektromagnetische Anordnung in Form von Wicklungen um das Aufnahmevolumen (2) herum und/oder um die zweite Einrichtung (16) herum vorgesehen ist.

9. Verabreichungsvorrichtung nach einem der Ansprüche 1 bis 8, **dadurch gekennzeichnet, dass** der Kolben (3) eine Ausnehmung (3a) aufweist, die die erste Einrichtung (15) wenigstens teilweise aufnimmt.

10. Verabreichungsvorrichtung nach einem der Ansprüche 1 bis 9, **dadurch gekennzeichnet, dass** ein Kraftsensor (14) vorgesehen ist, der in Axialrichtung der Kolbenstange misst.

11. Verabreichungsvorrichtung nach einem der Ansprüche 1 bis 10, **dadurch gekennzeichnet, dass** das Aufnahmevolumen (2) relativ zum Kolben (3) mit daran festgelegter Kolbenstange verstellbar ist.

12. Verabreichungsvorrichtung nach einem der Ansprüche 1 bis 10, **dadurch gekennzeichnet, dass** das Aufnahmevolumen austauschbar festgelegt ist, während der Kolben (3) über die Kolbenstange (6a) verstellbar ist.
